# EUROPEAN PATENT APPLICATION

(11) **EP 1 702 613 A1**
(43) Date of publication of application: **20.09.2006**
(21) Application number: 04807004.9
(22) Date of filing: 14.12.2004
(51) Int. Cl.: A61K 31/122, A61P 1/16, A61P 31/12, A61P 35/00

(54) **PREVENTIVE FOR THE ONSET OF LIVER CANCER COMPRISING QUINONE COMPOUND AS THE ACTIVE INGREDIENT**

(30) Priority: 06.01.2004 JP 2004001215
(71) Applicant: Eisai Co., Ltd., Tokyo 112-8088 (JP); Osaka Industrial Promotion Organization, Osaka-shi, Osaka 540-0029 (JP)
(72) Inventor: SHIOMI, Susumu, 5460037 (JP)
(74) Representative: HOFFMANN EITLE
(86) International application number: PCT/JP2004/018643
(87) International publication number: WO 2005/065671

(57) **Abstract**

The present invention provides a safe and effective drug to prevent carcinogenesis of liver cancer derived, above all, from hepatitis C virus cirrhosis. The present invention provides a preventive agent for carcinogenesis of liver cancer containing menatetrenone as an active ingredient, and is effective in preventing the carcinogenesis of liver cancer by administration of such a preventive agent.

## Description

### Technical Field

The present invention relates to a preventive agent for carcinogenesis of liver cancer containing a quinone-based compound as an active ingredient, and more particularly, the present invention relates to a preventive agent for carcinogenesis of liver cancer containing menatetrenone as an active ingredient.

### Background Art

Approximately 90% of patients with liver cancer in Japan are caused by the hepatitis viruses, most notably, approximately 70% of these patients are caused by the hepatitis C virus (for example, see Non-Patent Literature 1: Liver, Vol. 41, p799-811, 2000). The cumulative percentage of carcinogenesis in hepatitis C virus cirrhotic patients within 5 years is 27%, within 10 years is 52.4%, and within 15 years is 70.6% (for example, see Non-Patent Literature 2: Liver, Vol. 35, p772-773, 1994).

Interferon therapy, which has the object of combating the hepatitis virus, is well known as a therapy to prevent the transition from chronic viral hepatitis to liver cancer (for example, see Non-Patent Literature 3: Nishiguchi N. et al.: Lancet 346, p 1051-1055, 1995). However, it is known that interferon has problems with such side effects as interstitial pneumonia, depression and diabetes (for example, see Non-Patent Literature 4: Liver, Gall Bladder and Pancreas, Vol. 32, p101-127, 1996).

On the one hand, vitamin K is a coenzyme of γ-glutamyl carboxylase that carboxylates glutamate residues of a vitamin K dependent protein on an N terminal. Examples of vitamin K dependent proteins include blood coagulant factors II (prothrombin), VII, IX, X and the like. The vitamin K family comprises naturally occurring K1 and K2, as well as synthetic K3. Above all, vitamin K2 (generic term "menatetrenone") is marketed as a therapeutic drug for osteoporosis.

On the other hand, menatetrenone is known to have a differentiation induction activity in leukemia cells (for example, see Patent Literature 1: Japanese Unexamined Patent Application Publication No. H6-305955). However, the literature mentioned above describes only in vitro effects and does not describe clinical preventive effects on carcinogenesis of hepatic cells.

Moreover, geranylgeraniol is present on a side chain part of menatetrenone, and is also known to be a potent inducer of apoptosis in tumor cells (for example, see Non-Patent Literature 5: Ohizumi H., Masuda Y., Nakajo S., Sakai I., Ohsawa S., and Nakaya: Geranylgeraniol is a potent inducer of apoptosis in tumor cells. J. Biochem. 1995, 117:11-13). However, in the above cited literature, there is no suggestion of clinically preventing carcinogenesis in normal liver cells in the same way.

Further, recently it has become known that menatetrenone suppresses recurrence of liver cancer which has been treated, and heightens the survival percentage (for example, see Non-Patent Literature 6: Liver, Vol 43, suppl. (1), A64, 2002; and Non-Patent Literature 7: Proceedings of the 38th Japan Liver Cancer Research Society, p. 135, 2002). However, the aforementioned literatures target patients who have already contracted cancer, and do not indicate the carcinogenesis prevention effect for cirrhotic patients in whom no cancer has developed.

### Disclosure of Invention

### Problems to be Solved by the Invention

As mentioned above, currently, a safe and effective drug to prevent carcinogenesis of liver cancer has not yet been offered, and such a drug is desirable. Consequently, an object of the present invention is to provide a safe and effective drug to prevent carcinogenesis of liver cancer.

### Means to Solve the Problems

With the foregoing in view, as a result of assiduous study, the present inventor has completed the present invention by discovering that menatetrenone has a preventive effect of the carcinogenesis of liver cancer.

Specifically, in the first aspect, the present invention provides: 1) apreventive agent for carcinogenesis of liver cancer, comprising menatetrenone as an active ingredient; 2) apreventive agent for carcinogenesis of cancer derived from chronic liver disease, comprising menatetrenone as an active ingredient; 3) a preventive agent for carcinogenesis of liver cancer derived from cirrhosis, comprising menatetrenone as an active ingredient; 4) a preventive agent for carcinogenesis of liver cancer derived from hepatitis virus cirrhosis, comprising menatetrenone as an active ingredient; 5) a preventive agent for carcinogenesis of liver cancer derived from hepatitis C virus cirrhosis, comprising menatetrenone as an active ingredient.

In the second aspect, the present invention also provides: 6) a method for preventing carcinogenesis of liver cancer, comprising administering menatetrenone; 7) a method for preventing carcinogenesis of cancer derived from chronic liver disease, comprising administering menatetrenone; 8.) a method for preventing carcinogenesis of live cancer derived from chronic liver disease, comprising administering menatetrenone; 9) a method for preventing carcinogenesis of live cancer derived from hepatitis virus cirrhosis, comprising administering menatetrenone; 10) a method for preventing carcinogenesis of live cancer derived from hepatitis C virus cirrhosis, comprising administering menatetrenone.

In the third aspect, the present invention also provides: 11) a use of menatetrenone for the manufacture of a preventive agent for carcinogenesis of liver cancer; 12) a use of menatetrenone for the manufacture of a preventive agent for carcinogenesis of cancer derived from chronic liver disease; 13) a use of menatetrenone for the manufacture of a preventive agent for carcinogenesis of liver cancer derived from cirrhosis; 14) a use of menatetrenone for the manufacture of a preventive agent for carcinogenesis of liver cancer derived from hepatitis virus cirrhosis; 15) a use of menatetrenone for the manufacture of a preventive agent for carcinogenesis of liver cancer derived from hepatitis C virus cirrhosis.

The term "liver cancer" used in the present invention mainly means hepatocellular carcinoma. Further, the term "chronic liver disease" used in the present invention means hepatitis and cirrhosis. Furthermore, the term "cirrhosis" used in the present invention means diseases classified F4 in the Inuyama classification system. Moreover, the term "hepatitis virus cirrhosis" used in the present invention mainly means cirrhosis derived from the hepatitis B or hepatitis C viruses. In addition, the term "carcinogenesis" used in the present invention means initial detection of cancer cells in patients who have not developed a cancer yet.

### Advantageous Effects of the Invention

The preventive agent for carcinogenesis containing menatetrenone according to the present invention provides an extremely superior effect relating to the prevention of the carcinogenesis of liver cancer in cirrhosis patients, specifically, in patients with cirrhosis derived from the hepatitis C virus.

### Brief Description of Drawings

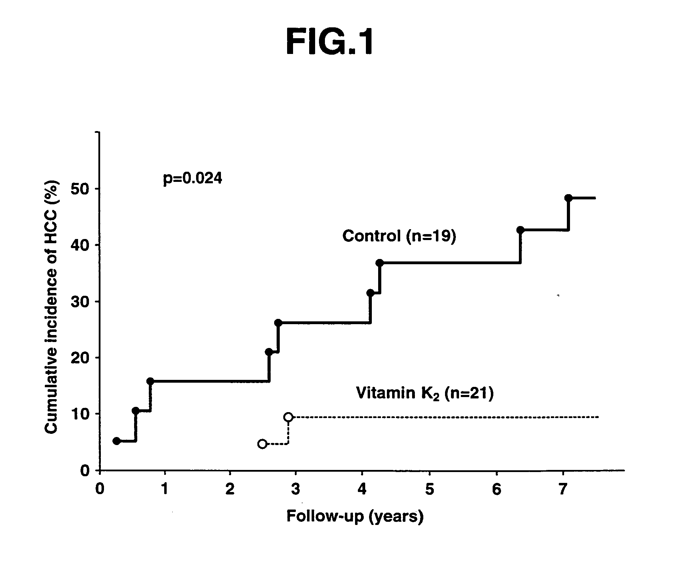

FIG. 1 indicates the cumulative incidence percentage of liver cancer. HCC in this figure is an abbreviation of "hepatocellular carcinoma".

### Best Mode for Carrying Out the Invention

The following embodiments are exemplary for explaining the present invention, and do not intend to limit the present invention to these embodiments. The present invention can be implemented in a variety of modes as long as there is no departure from the spirit and the scope of the present invention.

Menatetrenone used in the present invention has the chemical name 2-methyl-3-tetraprenyl-1,4-naphthoquinone. The structural formula of menatetrenone is indicated below.

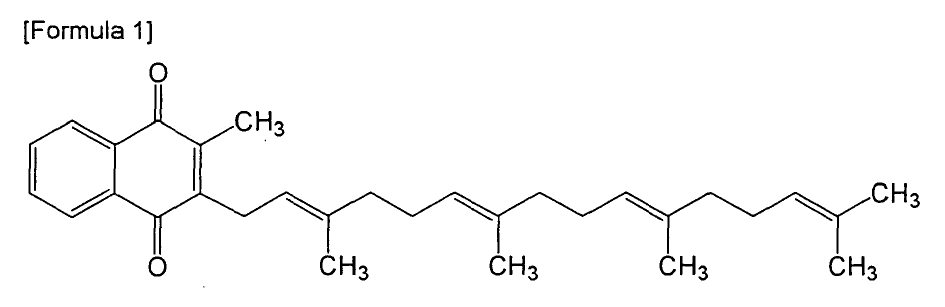

Menatetrenone is a yellow crystal or oily substance, odorless and tasteless, and is prone to photodegrade. Menatetrenone is also nearly insoluble in water.

Menatetrenone itself used in the present invention can be manufactured by well-known methods, and, as a representative example, is easily manufactured by the method disclosed in Japanese Patent Application Publication No. S49-55650; menatetrenone can also be readily available commercially from synthetic manufacturers.

Menatetrenone used in the present invention may be formed into an anhydride, or a hydrate. Moreover, menatetrenone is also present as a crystal polymorph, and may be a single crystal form or a mixture of crystal forms. Further, metabolites produced by the decomposition of menatetrenone in the living body are included in the scope of the claims of the present invention.

A medicament of the present invention may use menatetrenone as is, or menatetrenone may be manufactured using conventional methods by mixing with well-known pharmaceutically acceptable carriers (for example: fillers, binders, disintegrating agents, lubricants, colorants, flavorings and fragrances, stabilizers, emulsifiers, absorption promoters, surfactants, pH adjusters, preservatives, and antioxidants, etc.), and by mixing with components generally used as raw ingredients of pharmaceutical preparations. Moreover, components such as vitamins, and amino acids may be mixed as necessary. Examples of dosage forms for the pharmaceutical preparation include tablets, powders, fine granules, granules, capsules, syrups, suppositories, injectable agents, ointments, skin patches and the like.

In addition, the mode for administration of menatetrenone is not particularly limited in the present invention, but oral administration is preferable. Menatetrenone can be acquired as capsules under the commercial names of Kaytwo capsule (manufactured by Eisai Co., Ltd.) and Glakay capsule (manufactured by Eisai Co., Ltd.); as syrup under the commercial name of Kaytwo syrup (manufactured by Eisai Co., Ltd.); and as an injectable agent under the commercial name of Kaytwo N for injection (manufactured by Eisai Co., Ltd.).

In general, a dosage of an agent containing menatetrenone is preferably from 10 to 200 mg / day, and more preferably, from 30 to 135 mg / day.

Test examples of the present invention are described below, but these are exemplary, and the present invention is not limited to these test examples. In addition to the test examples indicated below, a person skilled in the art can make a variety of modifications within the scope of the present invention, and such modifications are also included in the scope of the claims of the present application.

### Test Example 1

### Method

A primary test was conducted targeting 50 female virus cirrhosis patients.

The diagnosis of cirrhosis was based on histological examination of liver specimens obtained by laparoscopy or needle biopsy performed under ultrasonic guidance.

If results of abdominal dynamic CT and abdominal ultrasonography suggested the presence of hepatocellular carcinoma, a liver biopsy was performed.

Half were randomly assigned to receive 45 mg/day of vitamin K2 (Glakay; Eisai Co., Tokyo, Japan) p.o., and half were assigned to the control group. Randomization was performed by placing the treatment assignments in sealed envelopes.

At the end of the first study, 21 patients in the treatment group and 19 patients in the control group agreed to participate in a longer trial. These patients were infected with the hepatitis C virus except for one patient in each group that had hepatitis B.

Seven patients, 4 in the control group and 3 in the treatment group, had previously been treated with interferon (IFN)-α for their HCV infection, but none had demonstrated a sustained response. Three patients in the earlier control group were found to have HCC during the study, even though careful examination at entry had not detected the lesion.

The clinical trial was conducted in accordance with the Helsinki Declaration and was approved by our ethics committee.

Statistical analysis was carried out with SAS software (version 8.12). The χ2 test was used to assess the homogeneity of groups. Cumulative incidences were plotted by the Kaplan-Meier method, and statistical significance of differences was analyzed by the log-rank test. Cox's regression analysis was used for univariate and multivariate analysis. In both studies, the two groups were similar with respect to age, the Child-Pugh stage, and the other clinical findings (See Table 1).

### Table 1

**Table 1. Baseline characteristics**

| | Treatment (n=21) | Control (n=19) | P value |
|---|---|---|---|
| Average age (yo) | 59.8 ± 8.7 | 61.4 ± 7.1 | ns |
| HBV/HCV | 1/20 | 1/18 | ns |
| Albumin (g/dl) | 3.9 ± 0.3 | 3.9 ± 0.3 | ns |
| Platelets (10⁴/mm³) | 14.7 ± 5.4 | 12.1 ± 5.2 | ns |
| Total bilirubin (mg/dl) | 0.8 ± 0.2 | 0.9 ± 0.4 | ns |
| ALT (IU/ml) | 81.7 ± 42.7 | 70.4 ± 33.4 | ns |
| AFP (µg/dl) | 13.4 ± 17.7 | 13.3 ± 8.7 | ns |

Mann-Whitney U test for age, serum albumin, platelets, total bilirubin, ALT and AFP; chi-square test for HBV / HCV.

### Results

As shown in FIG. 1, as results of a 7-year follow-up study, while the cumulative number of patients with the onset of liver cancer increased in the control group, ultimately leading to liver cancer in 9 of 19 patients, only 2 of 21 patients in the treatment group developed liver cancer. In this way, the cumulative incidence percentage of liver cancer patients in the treatment group was much smaller than that in the control group.

According to univariate analysis, odds ratio of the treatment group to the control group of developing liver cancer was 0.195 (from 0.042 to 0.913) (See Table 2).

### Table 2

**Table2. Crude Odds ratios for development of HCC**

| | Odds ratio | 95%CI | P-value |
|---|---|---|---|
| VK2/Control | 0.195 | 0.042-0.913 | 0.038 |
| Total bilirubin (1.0+/<1.0) | 1.231 | 0.326-4.643 | 0.760 |
| Serum albumin (<3.5/3.5+) | 3.800 | 0.800-18.459 | 0.093 |
| Platelet count (<100/100+) | 1.547 | 0.461-5.187 | 0.480 |
| ALT(<80/80+) | 0.624 | 0.165-2.356 | 0.487 |
| AFP(20+/<20) | 1.673 | 0.359-7.786 | 0.512 |
| IFN(+/-) | 1.000 | 0.215-4.646 | 1.000 |

When conducting multivariate analysis compensating for age, alanine aminotransferase activity (ALT), plasma albumin, total bilirubin, platelet count, α-fetoprotein (AFP), and history of treatment by IFN-α, the odds ratio of the treatment group to the control group of developing liver cancer was 0.126 (0.016 to 0.992) (See Table 3).

### Table 3

**Table3. Adjusted Odds ratios for development of HCC**

| | Odds ratio | 95%CI | P-value |
|---|---|---|---|
| VK2/Control | 0.126 | 0.016-0.992 | 0.0491 |
| Total bilirubin (1.0+/<1.0) | 0.294 | 0.042-2.044 | 0.2161 |
| Serum albumin (<3.5/3.5+) | 33.434 | 2.362-473.352 | 0.0094 |
| Platelet count (<100/100+) | 2.235 | 0.458-10.900 | 0.3200 |
| ALT(<80/80+) | 0.393 | 0.071-2.164 | 0.2831 |
| AFP(20+/<20) | 1.689 | 0.306-9.335 | 0.5477 |
| IFN(+/-) | 1.260 | 0.201-7.903 | 0.8053 |

Adjusted for age and all other variables in this table.

It should be noted that the term "CI" in Tables 2 and 3 is an abbreviation of Confidential Interval, and means the "confidence interval".

### Industrial Applicability

With the preventive agent for carcinogenesis containing menatetrenone as the active ingredient according to the present invention, superior effects in preventing liver cancer of patients suffering from cirrhosis, in particular, patients suffering from hepatitis C virus cirrhosis, can be exhibited.

## Claims

1. A preventive agent for carcinogenesis of liver cancer, comprising menatetrenone as an active ingredient.

2. A preventive agent for carcinogenesis of cancer derived from chronic liver disease, comprising menatetrenone as an active ingredient.

3. A preventive agent for carcinogenesis of liver cancer derived from cirrhosis, comprising menatetrenone as an active ingredient.

4. A preventive agent for carcinogenesis of liver cancer derived from hepatitis virus cirrhosis, comprising menatetrenone as an active ingredient.

5. A preventive agent for carcinogenesis of liver cancer derived from hepatitis C virus cirrhosis, comprising menatetrenone as an active ingredient.

6. A method for preventing carcinogenesis of liver cancer, comprising administering menatetrenone.

7. A method for preventing carcinogenesis of cancer derived from chronic liver disease, comprising administering menatetrenone.

8. A method for preventing carcinogenesis of live cancer derived from chronic liver disease, comprising administering menatetrenone.

9. A method for preventing carcinogenesis of live cancer derived from hepatitis virus cirrhosis, comprising administering menatetrenone.

10. A method for preventing carcinogenesis of live cancer derived from hepatitis C virus cirrhosis, comprising administering menatetrenone.

11. A use of menatetrenone for the manufacture of a preventive agent for carcinogenesis of liver cancer.

12. A use of menatetrenone for the manufacture of a preventive agent for carcinogenesis of cancer derived from chronic liver disease.

13. A use of menatetrenone for the manufacture of a preventive agent for carcinogenesis of liver cancer derived from cirrhosis.

14. A use of menatetrenone for the manufacture of a preventive agent for carcinogenesis of liver cancer derived from hepatitis virus cirrhosis.

15. A use of menatetrenone for the manufacture of a preventive agent for carcinogenesis of liver cancer derived from hepatitis C virus cirrhosis.
